# EUROPÄISCHE PATENTSCHRIFT

(11) **EP 1 146 049 B1**
(45) Veröffentlichungstag und Bekanntmachung des Hinweises auf die Patenterteilung: **17.08.2005**
(21) Anmeldenummer: 01113857.5
(22) Anmeldetag: 08.02.1995
(51) Int. Cl.: C07H 1/08, C07H 21/00, C12N 15/10, C12P 19/34, C12Q 1/68

(54) **Verfahren zur Trennung von Doppelstrang/Einzelstrangnukleinsäurestrukturen**
Process for separating double-stranded/single-stranded nucleic acid structures
Procédé de séparation de structures d'acides nucléiques à deux brins et à un brin

(30) Priorität: 11.02.1994 DE 4404361
(43) Veröffentlichungstag der Anmeldung: 17.10.2001
(62) Teilanmeldung aus: 95909684.3
(73) Patentinhaber: Qiagen GmbH, 40724 Hilden (DE)
(72) Erfinder: Bastian, Helge, 40822 Mettmann (DE); Feuser-Frank, Petra, 46509 Xanten (DE); Gauch, Simone, Pasadena, CA 91101 (US); Metin, Colpan, 45219 Essen (DE)
(74) Vertreter: Meyers, Hans-Wilhelm, Dr.Dipl.-Chem.

(56) Entgegenhaltungen:
- EP-A- 0 325 032
- EP-A- 0 389 063
- EP-A- 0 580 305
- WO-A-93/11221
- US-A- 5 075 430
- US-A- 5 155 018
- EGLY J.M. ET AL: "Separation of single-stranded from double-stranded nucleic acids using acriflavin-agarose chromatography" JOURNAL OF CHROMATOGRAPHY, Bd. 243, Seiten 301-306, XP001016042 AMSTERDAM (NL)

## Beschreibung

Gegenstand der vorliegenden Erfindung ist ein Verfahren zur chromatographischen Trennung von Nukleinsäuregemischen in ihre doppel- und einzelsträngigen Nukleinsäureanteile, indem Gesamtnukleinsäuren gleichzeitig an einen mineralischen Träger absorbiert werden und danach die Trennung in doppelsträngige Nukleinsäure und einzelsträngige Nukleinsäure durch fraktionierte Elution erfolgt oder indem doppelsträngige Nukleinsäure oder einzelsträngige Nukleinsäure einer flüssigen Probe selektiv an einen mineralischen Träger absorbiert werden sowie Lösungen und Kit zur Durchführung des erfindungsgemäßen Verfahrens.

Präparation von Nukleinsäuren, sowohl RNA wie auch DNA, hat zunehmend an Bedeutung gewonnen. Dabei werden beispielsweise die biologischen Quellen, aus denen die RNA oder DNA isoliert werden soll, aufgeschlossen, beispielsweise durch mechanische Einflüsse oder chemische Einflüsse, wie Behandlung mit Detergenzien etc. So folgt auf den Zellaufschluß zur Gewinnung der Nukleinsäure üblicherweise eine Cäsiumchlorid-Dichtegradientenzentrifugation oder eine Extraktion mit Phenol. Diese Methoden sind zwar zur Isolation von Nukleinsäuren geeignet, weisen aber Nachteile auf, die ihre Handhabung erschweren. So setzt die Cäsiumchlorid-Dichtegrandientenzentrifugation den Einsatz von zeit- und kostenintensiver Ultrazentrifugation voraus, wohingegen der Umgang mit Phenol aus arbeitsschutzrechtlichen Erwägungen nicht unbedenklich erscheint.

So hat es in der Vergangenheit nicht an Versuchen gefehlt, die Isolation von Nukleinsäuren zu vereinfachen.

Die DE 36 39 949 A1, DE 40 34 036 A1 oder DE 41 39 664 A1 befassen sich z.B. mit Verbesserungen der Nukleinsäurereinigung mittels chromatographischer Methoden unter Vermeidung apparativ aufwendiger Verfahren, wie der Hochdruckflüssigkeitschromatographie (HPLC). Obwohl diese Methoden bereits beispielsweise gegenüber der Ultrazentrifugation oder Phenolextraktion einen Fortschritt darstellen, sind sie technisch relativ aufwendig und arbeitsintensiv. Da zur Fraktionierung oftmals mehrere Reinigungsschritte nacheinander durchgeführt werden müssen, ist die Bearbeitung insbesondere von kleinen Probenmengen z. B. durch Substanzverlust problematisch.

Die EP 0 389 063 A2 betrifft ebenfalls ein Verfahren zur Isolierung von Nukleinsäuren. Dabei wird die Nukleinsäuren enthaltende Quelle in Gegenwart chaotroper Ionen aufgeschlossen und dann mit einem unter diesen Bedingungen Nukleinsäuren adsorbierendem Material behandelt. Als solches Material wird Diatomeenerde oder andere Siliciumoxid enthaltende mineralische Träger genannt. Es gelingt nach der in der EP 0 389 063 A2 genannten Methode RNA und DNA und RNA und ssRNA gleichzeitig zu isolieren. Eine wünschenswerte Fraktionierung der an dem Siliciumdioxidträger gebundenen Nukleinsäuren in DNA- und RNA-Anteile wird jedoch nicht erreicht. RNA kann dann durch Zugabe von RNAse abgebaut werden, so daß die DNA übrig bleibt.

Gillespie et al. offenbaren im US-Patent-Nr. US 5,155,018 ein Verfahren zur Isolierung und Reinigung von biologisch aktiver RNA aus biologischen Quellen enthaltend RNA, DNA und andere Zellinhaltsstoffe. Dabei wird die RNA enthaltende Quelle mit Partikeln in Kontakt gebracht, welche silicagelhaltige Materialien wie feinverteiltes Glas sind. Der Bindungspuffer, aus welchem die RNA an dem Material adsorbiert wird, ist angesäuerte chaotrope Salze enthaltende Lösungen. Unter diesen Bedingungen wird RNA aber nicht DNA an dem Silicamaterial gebunden. Die Verwendung von angesäuerten chaotropen Puffern besitzt den Nachteil, daß bei der Ansäuerung Guanidiniumthiocyanat (GTC)-haltiger Bindungspuffer die Gefahr der Blausäurebildung besteht und somit besondere Vorsichtsmaßnahmen getroffen werden müssen. Weiterhin wird die DNA durch Säureeinwirkung zerstört. Darüberhinaus läßt sich nach diesem Verfahren eine DNA Aufreinigung aus der authentischen Probe nicht durchführen.

Little beschreibt in US-Patent-Nr. 5,075,430 ein Verfahren zur Reinigung von Plasmid- und anderer DNA, sowohl einzelsträngig wie auch doppelsträngig, durch Immobilisierung der DNA auf Diatomeenerde in Anwesenheit eines chaotropen Agens, woraufhin die DNA mit Wasser oder mit einem Puffer mit niedrigem Salzgehalt eluiert wird. Nach der Methode ist eine Reinigung von DNA/RNA nicht möglich.

M. A. Marko et al. beschreiben in "Analytical Biochemistry" 121, Seiten 382 bis 387 (1982) ein Verfahren zur Isolierung im großen Maßstab von hochgereinigter Plasmid-DNA unter Verwendung der alkalischen Extraktion und Bindung an Glaspulver. Eine Fraktionierung und getrennte Reinigung von RNA und DNA aus einer einzigen Probe wird nicht beschrieben.

An die Rohpräparation der Nukleinsäuren schließen sich Folgereaktionen an. Diese Folgereaktionen stellen bestimmte Anforderungen sowohl an die Isolierungsprozedur, als auch an die Reinheit und Integrität der isolierten Nukleinsäuren. Insbesondere wenn in der Folge enzymatische Amplifikationsreaktionen wie PCR (Polymerase Chain Reaction), LCR (Ligase Chain Reaction), NASBA (Nucleic Acid Sequence-Based Amplification) oder 3SR (self-sustained Sequence Replication) Anwendung finden, sollte die Präparation der Nukleinsäuren ohne die Gefahr von Kreuzkontaminationen anderer Proben möglich sein, und die isolierten Nukleinsäuren sollten frei von störenden Zellbestandteilen und/oder Metaboliten vorliegen. Die enzymatische Amplifikation von DNA (z. B. PCR) oder RNA (z. B. RNA-PCR) gewinnt aufgrund ihrer Spezifität und Sensitivität nicht nur in der Grundlagenforschung, sondern zunehmend auch im medizinischen Bereich für diagnostische Zwecke an Bedeutung. So beispielsweise für die Detektion von Nukleinsäuresequenzen aus kleinsten Mengen an Zellen und/oder Geweben oder Biopsiematerialien oder zum Nachweis von viralen Nukleinsäuren aus Blut oder Plasma. Für diese Anwendungen sind neben den genannten Anforderungen auch an Ausbeute und Reproduzierbarkeit des Verfahrens zur Nukleinsäureisolierung höchste Anforderungen gestellt.

Ein der Erfindung zugrundeliegendes technisches Problem besteht darin, ein Verfahren anzugeben, mit dem es nicht nur gelingt RNA und DNA aus biologischen Proben wie Zell-Lysaten und Gewebe-Lysaten getrennt voneinander aber aus derselben Probe stammend aufzureinigen, sondern generell doppelsträngige von einzelsträngige Nukleinsäuren. Das Verfahren sollte dabei möglichst kostengünstig arbeiten, indem beispielsweise preisgünstige unmodifizierte Trennmaterialien Verwendung finden können. Das Verfahren sollte darüber hinaus auch zur Probenvorbereitung für die Diagnostik geeignet und mit verschiedenen Amplifikationsmethoden kompatibel sein. Weiterhin sollen die bei der Diskussion des Standes der Technik angesprochenen Nachteile vermieden werden.

Überraschenderweise wird das der Erfindung zugrundeliegende technische Problem durch ein Verfahren mit den Merkmalen des Patentanspruchs 1 in seinen Verfahrensalternativen 1.1 bis 1.4 gelöst. Die Unteransprüche 2 bis 11 betreffen bevorzugte Ausführungsformen des erfindungsgemäßen Verfahrens, die Ansprüche 12 bis 21 Lösungen zur Verwendung im erfindungsgemäßen Verfahren bzw. die Verwendung dieser Lösungen und Anspruch 22 betrifft eine Zusammenstellung enthaltend die für das erfindungsgemäße Verfahren notwendigen Komponenten.

Im einzelnen stellt sich das erfindungsgemäße Verfahren zur Fraktionierung von doppelsträngigen und einzelsträngigen Nukleinsäure-Strukturen aus biologischen Quellen in folgenden Verfahrensalternativen dar.

Die zu trennende Nukleinsäurearten (einzel- und doppelsträngige) enthaltende Probe wird mit mindestens einem mineralischen Träger behandelt, wobei die Behandlungsbedingungen durch ein entsprechendes wäßriges Gemisch von Salzen, insbesondere chaotrope Substanzen und Alkoholgruppen enthaltender Substanz so eingestellt sind, daß überwiegend die einzelsträngige Nukleinsäure-Fraktion an einem ersten mineralischen Träger adsorbiert wird, während die doppelsträngige Nukleinsäure nicht adsorbiert wird. Die austretende doppelsträngige Nukleinsäure kann dann mit an sich bekannten Verfahren weiter bearbeitet werden. Die am ersten mineralischen Träger adsorbierte einzelsträngige Nukleinsäure wird nach gegebenenfalls durchgeführten Waschschritten eluiert unter Bedingungen geringer Ionenstärke oder mit Wasser. Die nicht adsorbierte doppelsträngige Nukleinsäure, die aufgefangen wurde, kann weiter aufgereinigt werden, indem z.B. die Fraktion anschließend durch ein entsprechendes wäßriges Gemisch von Salzen, insbesondere chaotrope Substanzen, und alkoholgruppenhaltigen Substanzen auf solche Bedingungen eingestellt wird, daß die doppelsträngige Nukleinsäure an einem zweiten mineralischen Träger adsorbierbar und, nach gegebenenfalls durchgeführten Waschschritten, eluierbar wird unter Bedingungen geringer Ionenstärke oder mit Wasser.

In einer zweiten Ausführungsform des erfindungsgemäßen Verfahrens werden zur Trennung von einzelsträngiger Nukleinsäure und doppelsträngiger Nukleinsäure die Behandlungsbedingungen so eingestellt, daß Erdalkali-Ionen komplexierende Substanzen in Abwesenheit von Alkoholgruppen enthaltenden Substanzen in der Lösung enthalten sind, wobei die einzelsträngige Nukleinsäure nicht an dem ersten mineralischen Träger adsorbiert wird und von der übrigen Probe abgetrennt werden kann. Die abgetrennte einzelsträngige Nukleinsäure kann dann mit an sich bekannten Verfahren weiter bearbeitet werden. Die doppelsträngige Nukleinsäure bindet jedoch überwiegend an dem ersten mineralischen Träger und kann, nach gegebenenfalls durchgeführten Waschschritten, eluiert werden, unter Bedingungen geringer Ionenstärke oder mit Wasser. Die so erhaltene doppelsträngige Nukleinsäure kann dann mit an sich bekannten Verfahren weiter bearbeitet werden.

Die nicht adsorbierte einzelsträngige Nukleinsäure, welche aufgefangen wurde, kann dann anschließend insbesondere durch Zugabe von Alkoholgruppen enthaltenden Substanzen auf solche Bedingungen eingestellt werden, daß die einzelsträngige Nukleinsäure dann an einem zweiten mineralischen Träger adsorbierbar und, nach gegebenenfalls durchgeführten Waschschritten, eluierbar wird unter Bedingungen geringer Ionenstärke oder mit Wasser.

Werden die Behandlungsbedingungen so eingestellt, daß Netz-, Wasch- oder Dispergiermittel in Abwesenheit von Alkoholgruppen aufweisenden Substanzen in der Lösung enthalten sind, wird die einzelsträngige Nukleinsäure unter diesen Behandlungsbedingungen nicht an einem ersten mineralischen Träger adsorbiert und kann mithin von der übrigen Probe abgetrennt und weiter verarbeitet werden. Die doppelsträngige Nukleinsäure bindet jedoch überwiegend an den ersten mineralischen Träger und kann nach gegebenenfalls durchgeführten Waschschritten unter Bedingungen geringer Ionenstärke oder mit Wasser eluiert werden. Die eluierte doppelsträngige Nukleinsäure kann danach mit an sich bekannten Verfahren weiter bearbeitet werden.

Die nicht adsorbierte, aufgefangene einzelsträngige Nukleinsäure kann dann anschließend vorzugsweise durch Zugabe von Alkoholgruppen enthaltenden Substanzen auf solche Bedingungen eingestellt werden, daß die einzelsträngige Nukleinsäure dann an einem zweiten mineralischen Träger adsorbierbar und, nach gegebenenfalls durchgeführten Waschschritten, eluierbar wird unter Bedingungen geringer Ionenstärke oder mit Wasser.

Eine weitere Ausführungsform des erfindungsgemäßen Verfahrens gewährleistet die Fraktionierung von gemeinsam gebundener einzelsträngiger Nukleinsäure und doppelsträngiger Nukleinsäure. Dabei werden die Behandlungsbedingungen durch ein entsprechendes wäßriges Gemisch von Salzen, insbesondere chaotrope Substanzen und Alkoholgruppen enthaltenden Substanzen so eingestellt, daß die Gesamtnukleinsäure aus einzelsträngiger Nukleinsäure und doppelsträngiger Nukleinsäure an einem mineralischen Träger adsorbiert wird, gefolgt von einer Fraktionierung der an den ersten Träger gebundenen doppelsträngigen/einzelsträngigen Nukleinsäure durch selektive Elution der doppelsträngigen Nukleinsäure mittels Behandlung mit einer Lösung verminderter Ionenstärke und Konzentration einer Alkoholgruppen enthaltenden Substanz oder Elution der einzelsträngigen Nukleinsäure mittels einer Lösung enthaltend eine Erdalkali-Ionen komplexierende Substanz und/oder ein Netz-, Wasch- oder Dispergiermittel sowie eine oder mehrere Salzart(en), insbesondere chaotrope Substanzen. Im ersten Fall bleibt dann die einzelsträngige Nukleinsäure auf dem Träger gebunden, wohingegen im zweiten Falle die doppelsträngige Nukleinsäure an dem mineralischen Träger gebunden bleibt. Die jeweils eluierte Fraktion kann dann mit an sich bekannten Verfahren weiter bearbeitet werden.

Die Einstellung der Behandlungsbedingungen mit Alkoholgruppen enthaltenden Substanzen und Salzen, insbesondere chaotrope Substanzen zur Trennung der Nukleinsäuren erfolgt erfindungsgemäß unter Zugrundelegung der nachstehenden physiko-chemischen Grundlagen, die hier zum ersten Mal formuliert sind.

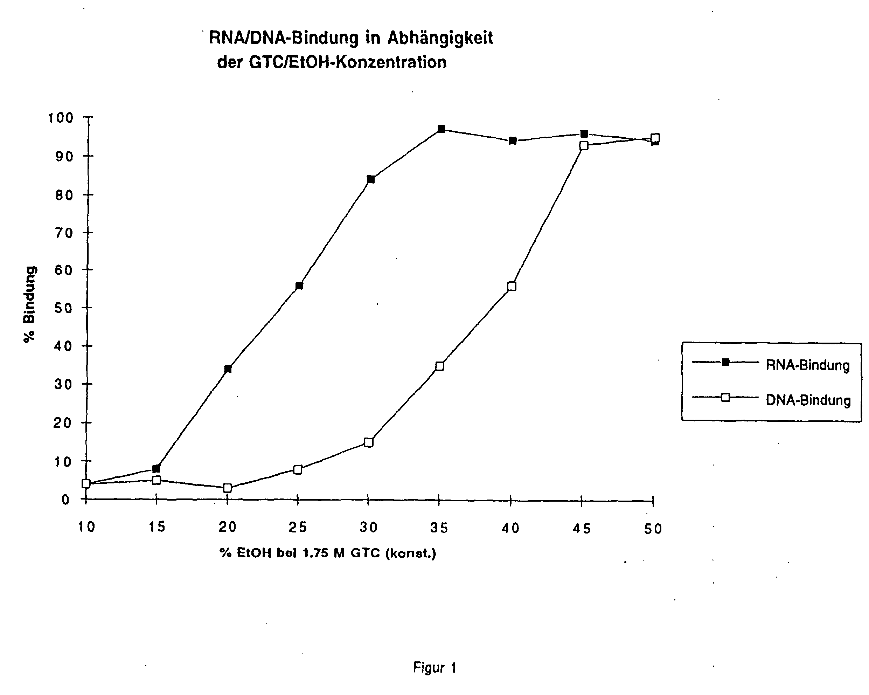

Fig. 1 zeigt die Bindung einzelsträngiger/doppelsträngiger Nukleinsäure am Beispiel einzelsträngiger RNA und doppelsträngiger DNA. Es wird die RNA/DNA-Bindung aus einem Gewebelysat an einen mineralischen Träger in Abhängigkeit der Konzentration einer Alkoholgruppen enthaltenden Substanz (hier Ethanol) und einer chaotropen Substanz (hier GTC) beschrieben. Unter der Voraussetzung, daß die Konzentration einer der Substanzen, Alkohol oder chaotroper Substanz, konstant ist, ist festzustellen, daß bei hoher Alkoholkonzentration und/oder Menge chaotroper Substanz, beide Nukleinsäurearten (RNA/DNA) am mineralischen Träger gebunden werden. Unterschreitet die Konzentration einer oder beider Substanzen (Alkohol oder chaotropes Reagenz) einen bestimmten Wert, dann bindet keine der Nukleinsäure in nennenswertem Maße an dem mineralischen Träger. Dazwischen binden überraschenderweise RNA und DNA so unterschiedlich an den mineralischen Träger, daß dies zur Trennung der Nukleinsäuren ausgenutzt werden kann. So können - ausgehend von Zellen - nach Lyse der Zellen mit hoher Konzentration an chaotropen Substanzen, durch anschließende Zugabe einer Alkoholgruppen enthaltenden Substanz oder eines Gemisches aus Alkoholgruppen enthaltender Substanz und Wasser oder Puffer, Konzentrationen von chaotroper Substanz und Alkoholgruppen enthaltener Substanz so eingestellt werden, daß eine selektive Bindung der RNA erzielt wird, während die DNA im Durchbruch verbleibt. Im Beispiel gemäß Figur 1 würde man eine Konzentration von 1,75 M GTC und 30 Vol% Ethanol wählen, um eine Trennung von RNA von DNA durch fraktionierte Bindung zu erreichen.

Andererseits kann unter Bedingungen hoher Konzentration an Alkoholgruppen enthaltender Substanz und/oder hoher Konzentration an chaotroper Substanz eine simultane Bindung von einzelsträngiger Nukleinsäure und doppelsträngiger Nukleinsäure am mineralischen Träger erreicht werden, und durch Verringerung der Konzentration an Alkoholgruppen enthaltender Substanz und/oder chaotroper Substanz zunächst die Desorption der doppelsträngigen Nukleinsäure eingeleitet werden. Die einzelsträngige Nukleinsäure bleibt dabei gebunden und eluiert bei noch weiterer Verringerung der Konzentration einer oder beider Substanzen. Im Beispiel gemäß Fig. 1 würde man Konzentrationen von 1,75 M GTC und 45 Vol% Ethanol wählen, um eine Bindung der Gesamtnukleinsäure zu erreichen. Wie in Beispiel 8 exemplarisch verdeutlicht wird, würde man zur selektiven Desorption der DNA eine Konzentration von 0,3 M GTC und 10 Vol% Ethanol wählen.

Es ist mithin möglich, die RNA und DNA durch Adsorption an einem mineralischen Träger zu trennen, oder aber zunächst die Gesamtnukleinsäure an den mineralischen Träger zu adsorbieren und die einzelsträngige Nukleinsäure oder doppelsträngige Nukleinsäure selektiv zu eluieren.

Gegebenenfalls können vor der Elution der jeweiligen Nukleinsäure (einzelsträngige Nukleinsäure oder doppelsträngige Nukleinsäure) auch Waschschritte durchgeführt werden.

Die Elution erfolgt dann jeweils unter Bedingungen geringer Ionenstärke oder mit Wasser. Die zuerst vom mineralischen Träger desorbierte Nukleinsäure wird anschließend durch Erhöhung der Ionenstärke und/oder der Konzentration Alkoholgruppen enthaltender Substanzen so eingestellt, daß die doppelsträngige Nukleinsäure oder einzelsträngige Nukleinsäure an einem zweiten mineralischen Träger adsorbiert wird und, nach gegebenenfalls durchgeführten Waschschritten, eluiert wird unter Bedingungen geringer Ionenstärke oder mit Wasser.

Da Nukleinsäuren beispielsweise auch in Kochsalz/Ethanol-Gemischen an mineralische Träger adsorbieren und unter Bedingungen geringer Ionenstärke oder mit Wasser eluiert werden können, ist anzunehmen, daß die im erfindungsgemäßen Verfahren verwendeten Salzlösungen nicht zwingend chaotrope Salze enthalten müssen, sondern daß jede Salzlösung in Kombination mit einer Alkoholgruppen enthaltenden Substanz Anwendung finden kann.

Das erfindungsgemäße Verfahren ermöglicht in vorteilhafter Weise die Bearbeitung kleiner Probenmengen, gewährleistet eine einfache und sichere Handhabung unter Vermeidung von Präzipitationsschritten. Weiterhin ist das Verfahren gemäß der Erfindung wenig kosten- und personalintensiv durchführbar und kann in einfacher Weise die Bearbeitung einer Vielzahl von Proben gleichzeitig ermöglichen. Das Verfahren ist aufgrund seiner Vielseitigkeit und einfachen Handhabbarkeit auch für die Automatisierung geeignet.

Erfindungsgemäß können mit dem Verfahren aus Nukleinsäurestrukturen enthaltenden Quellen Doppelstrang/Einzelstrang-Nukleinsäurestrukturen getrennt werden. Als Quellen, die zu trennenden Nukleinsäurestrukturen beinhalten können, kommen die z.B. im Anspruch 7 genannten Quellen in Frage. Diese sind im einzelnen Zellkulturen, Gewebe jeder Art, Körperflüssigkeiten wie Blut, Plasma, Serum, Urin, Faeces, Mikroorganismen wie Bakterien, Viren wie Cytomegalie-Virus, HIV, Hepatitis B, Hepatitis C, Hepatitis-δ-Virus, Pflanzen, Pflanzenteile, Embryonen, Keimlinge, Früchte oder Nukleinsäuren enthaltende Gemische nach enzymatischen Reaktionen wie in vitro Transkription und/oder cDNA-Synthese und/oder reverse Transkription mit anschließender Polymerasekettenreaktion (PCR).

Zellen werden vorzugsweise zunächst in einem wäßrigen Lyse-system, welches chaotrope Substanzen und/oder andere Salze enthält, aufgeschlossen, indem im einfachsten Falle die Zellen damit versetzt werden. Gegebenenfalls kann durch mechanische Einwirkung der Prozeß des Aufschliessens beschleunigt werden.

Danach wird die so behandelte Probe, je nach Problemstellung welche Nukleinsäureart von der anderen getrennt werden soll, wie in den Verfahrensschritten 1.1 bis 1.4 gemäß Patentanspruch 1 beschrieben, weiter aufgearbeitet.

Einige der genannten Ausgangsmaterialien wie beispielsweise Bakterien können, aufgrund der Beschaffenheit ihrer Zellwände, nicht direkt in chaotrope Substanzen enthaltenden wäßrigen Systemen lysiert werden. Diese Ausgangsmaterialien müssen daher, bevor sie in dem erfindungsgemäßen Verfahren eingesetzt werden können, vorbehandelt werden, beispielsweise mit lytischen Enzymen.

Systeme zur Lysierung der die Nukleinsäure enthaltenden Quellen sind vorzugsweise Lösungen chaotroper Substanzen in einer Konzentration von 0,1 bis 10 M. Als chaotrope Substanzen kommen insbesondere Salze wie Natriumperchlorat, Guanidiniumhydrochlorid, Guanidinium-iso-thiocyanat/Guanidinium-thiocyanat, Natriumjodid, Kaliumjodid und/oder Kombinationen davon in Frage.

Auch wäßrige Lösungen enthaltend Salze wie Natriumchlorid, Lithiumchlorid, Kaliumchlorid, Natriumacetat, Magnesiumchlorid in einer Konzentration von 0,1 bis 10 M oder Harnstoff in entsprechenden Konzentrationen von 0,1 bis 10 M und/oder Kombinationen dieser Stoffe können als wäßrige Systeme zur Lyse bzw. Bindung der die Nukleinsäure enthaltenden Quellen verwendet werden.

Die Alkoholgruppen aufweisenden Substanzen sind vorzugsweise niedere aliphatische Alkohole mit 1 bis 5 Kohlenstoffatomen wie Methanol, Ethanol, Isopropanol, Butanol und Pentanol. Sie werden vorzugsweise in einer Konzentration von 1 bis 90 Vol.-% eingesetzt.

Der mineralische Träger besteht vorzugsweise aus porösen oder nicht porösen Metalloxiden oder Metallmischoxiden, Silicagel, Materialien, die überwiegend aus Glas bestehen, wie nicht modifizierte Glaspartikel, Glasmehl, Quarz, Aluminiumoxid, Zeolithe, Titandioxid, Zirkondioxid mit einer Partikelgröße des mineralischen Trägermaterials von 0,1 µm bis 1.000 µm und einer Porengröße von 2 bis 1.000 µm. Das poröse oder nicht poröse Trägermaterial kann in Form loser Schüttungen vorliegen oder als Filterschichten ausgebildet sein in Form von Filterschichten aus Glas, Quarz oder Keramik und/oder einer Membran, in der Silicagel angeordnet ist und/oder als Partikel oder Fasern aus mineralischen Trägern und Geweben aus Quarz oder Glaswolle vorliegen sowie Latex-Partikeln mit oder ohne funktionellen Gruppen oder Frittenmaterialien aus Polyethylen, Polypropylen, Polyvinyliden-fluorid, insbesondere ultra high molecular weight Polyethylen, high density Polyethylen.

Als Erdalkali-Ionen bindende Substanz kommt insbesondere Ethylendiamintetraessigsäure (EDTA) oder EGTA in Frage und als Netz-, Wasch- oder Dispergiermittel ist ein Sarkosinat einsetzbar.

Gewünschtenfalls können die erfindungsgemäß erhaltenen Nukleinsäuren durch weitere chromatographische Verfahren wie Anionenaustauscherchromatographie gereinigt werden.

Im erfindungsgemäßen Verfahren kann als einzelsträngige Nukleinsäure insbesondere RNA von doppelsträngiger Nukleinsäure (DNA) getrennt werden. Liegt DNA einzelsträngig vor, kann diese DNA dann auch von doppelsträngiger DNA, wie auch von doppelsträngiger RNA getrennt werden.

Die im erfindungsgemäßen Verfahren zur Anwendung kommenden Lösungen sind ebenfalls Gegenstand der vorliegenden Erfindung. Als Lysepuffer und/oder Bindungspuffer kommen erfindungsgemäß insbesondere wäßrige Lösungen enthaltend 0,5 bis 8,0 M Guanidinium-iso-thiocyanat/Guanidiniumthiocyanatund/oder Guanidinhydrochlorid, 0 bis 50 % Ethanol und/oder Isopropanol in Betracht.

Als Lösung zum Auswaschen bzw. Eluieren von an dem mineralischen Träger gebundenen Nukleinsäuren kommt eine wäßrige Lösung enthaltend 0,1 bis 3 M Guanidinium-iso-thiocyanat/Guanidiniumthiocyanat und/oder Guanidinhydrochlorid zusammen mit 1 bis 30 Vol.% Ethanol und/oder Isopropanol in Frage.

Als wäßriges Lösungssystem, das zum Binden von doppelsträngiger Nukleinsäure an mineralische Träger verwendet werden kann, kommt eine wäßrige Lösung enthaltend 1 bis 5 M Guanidin-iso-thiocyanat und/oder 1 bis 8 M Guanidinhydrochlorid mit 0,1 bis 5 % Sarkosinaten oder 5 mM bis 200 mM EDTA in Betracht. Zum Binden von doppelsträngiger Nukleinsäure kommt auch eine Lösung enthaltend 1 bis 5 M Guanidiniumthiocyanat und/oder 1 bis 8 M Guanidiniumhydrochlorid, 5 mM bis 200 mM EDTA oder EGTA in Frage.

Die erfindungsgemäß beanspruchte Zusammenstellung von Komponenten zur Durchführung des Verfahrens in einem Kit weist insbesondere zum Durchfluß geeignete Hohlkörper auf, in dem der oder die mineralische(n) Träger in der bereits weiter oben beschriebenen Form angeordnet ist (sind). Der mineralische Träger kann in loser Schüttung vorliegen, welche zwischen zwei Einrichtungen fixiert ist oder in Form von Membranen, die in dem Hohlkörper angeordnet sind. Desweiteren können in dem Kit Lösungen enthalten sein oder die Bestandteile für die Zusammenstellung der Lösungen in konzentrierter Form. Daraus kann dann der Anwender die jeweils benötigten Lösungen in der notwendigen Konzentration herstellen.

Ein weiterer vorteilhafter Bestandteil der Zusammenstellung ist eine Vorrichtung zur Homogenisierung der Lösung auf Zell-Lysaten. Eine besonders bevorzugte Vorrichtung zur Homogenisierung ist in der internationalen Patentanmeldung PCT/EP 95/00037 vorgeschlagen. Diese Vorrichtung besteht im wesentlichen aus mindestens zwei porösen Schichten, wobei die porösen Schichten abnehmende Porengröße aufweisen in Fließrichtung durch die Schichten gesehen. Beim Durchtritt des Zell-Lysats durch die abnehmende Porengrößen werden die viskosen Lösungen des Zell-Lysats homogenisiert.

Die Erfindung wird an den nachfolgenden Beispielen näher erläutert.

### Materialien und Methoden

### 1. Silica-Trägermaterialien

Silica-Trägermaterialien wurden in Membranform oder als suspendierte Partikel eingesetzt.

### 1.1. Silica-Membranen

Zwei Lagen einer Silica-Membran (z.B. Glasfaserfilter der Firma Whatman) wurden, wie in P 43 21 904 beschrieben, in einer Zentrifugen-Chromatographiesäule ("spin-Säule") fixiert. Für membranförmige Trägermaterialien wurde nach dem Standardprotokoll "spin-Prozedur" (vgl. 4.1) vorgegangen.

### 1.2. Silica-Partikel

Verschiedene Silica-Partikel (z.B. der Firmen Merck, Darmstadt und Sigma, St. Louis) wurden als 50 %-Suspension im jeweils verwendeten Lysepuffer (vgl. 3.1) eingesetzt. Die mittleren Partikeldurchmesser betrugen je nach Material 0,5 bis 50 µm. Es wurde nach dem Standardprotokoll "batch-Prozedur" (vgl. 4.2) vorgegangen.

### 2. Nukleinsäure enthaltende Quellen

### 2.1 Gewebe

Die zur Präparation eingesetzten Gewebe wurden sofort nach Entnahme in flüssigem Stickstoff gefroren und bei - 70°C gelagert. Alternativ kann frisches Gewebe verwendet werden.

### 2.2. Pflanzen

Blätter wurden unter flüssigem Stickstoff im Mörser zu einem feinen Pulver zermahlen und direkt zur Präparation eingesetzt oder bei - 70°C gelagert.

### 2.3. Zellkultur

Zellen wurden nach der Ernte zweimal mit PBS gewaschen und pelletiert. Aliquots mit entsprechender Zellzahl (bestimmt durch Auszählen in Thoma-Kammer) wurden frisch zur Präparation eingesetzt oder bei - 20°C gelagert.

Adhärent wachsende Zellen können alternativ in der Kulturschale gewaschen und durch Zugabe des jeweiligen Lysepuffers (vgl. 3.1) direkt in der Kulturschale lysiert werden.

### 2.4. Plasma

ACD-Blut wurde 10 min bei 3.000 x g zentrifugiert, der Überstand abgenommen und erneut wie oben zentrifugiert. Der nach der zweiten Zentrifugation erhaltene Überstand wurde aliquotiert und bei - 70°C gelagert.

### 2.5. Bakterien

Kulturen wurden mit einer Übernacht-Kultur angeimpft und bis zu einer OD₆₀₀ von 0,5 - 0,8 angezogen. Aliquots mit der entsprechenden Zellzahl (1 OD₆₀₀ = 10⁹ Zellen/ml) wurden pelletiert und die Zellpellets bei - 20°C gelagert oder frisch zur Präparation eingesetzt.

### 3. Reagenzien

### 3.1. Lysepuffer

- L1: 4,5 M GTC, 25 mM NaCitrat Ph 7,5, 0,7 % β-Mercaptoethanol (MSH)
- L2: 4,0 M GTC, 25 mM NaCitrat pH 7,5, 0,7 % β-MSH
- L3: 5,0 M GTC, 50 mM TRIS/HCl pH 7,0
- L4: 3,5 M GTC, 25 mM NaCitrat pH 7,5, 1 % β-MSH
- L5: 2,5 M GTC, 25 mM NaCitrat pH 7,5, 1 % β-MSH, 30 % Ethanol
- L6: 8,0 M GuHCl, 20 mM MOPS pH 7,0, 0,7 % β-MSH
- L7: 3,0 M GTC, 25 mM NaCitrat pH 7,5, 1 % β-MSH
- L8: 4,0 M GTC, 50 mM TRIS/HCl pH 7,5, 1 % Sarkosyl
- L9: 4,0 M GTC, 50 mM TRIS/HCl pH 7,5, 25 mM EDTA

### 3.2. Bindungsreagenz

- B1: Ethanol
- B2: n-Butanol
- B3: Isopropanol
- B4: 70 % Ethanol in Wasser
- B5: 5,9 M GTC

### 3.3. Waschpuffer

- W1: 2,0 M GTC, 25 mM TRIS/HCl pH 7,5, 30 % Ethanol
- W2: 4,0 M GTC, 40 mM TRIS/HCl pH 7,5, 20 % Isopropanol
- W3: 1,0 M GTC, 25 mM TRIS/HCl pH 7,5, 20 % Ethanol
- W4: 5,0 M GuHCl, 15 mM MOPS pH 7,0, 37 % Ethanol
- W5: 0,5 M GTC, 25 mM TRIS/HCl pH 7,5, 10 % Ethanol

### 4. Standardprotokolle

### 4.1. "spin-Prozedur"

1) Nukleinsäure enthaltende Quelle mit Lysepuffer (vgl. 3.1.) versetzen und mittels eines Handhomogenisators vollständig homogenisieren
2) Bindungsreagenz (vgl. 3.2.) zugeben, um die jeweiligen Bindebedingungen einzustellen
3) Lysat auf die spin-Säule pipettieren und 15 Sekunden bei 10.000 Upm in einer Tischzentrifuge durch die Membran der spin-Säule zentrifugieren; falls das Volumen des Lysates das Füllvolumen der spin-Säule überschreitet, diesen Bindungsschritt wiederholen
4) den Säulendurchbruch gegebenenfalls weiteraufarbeiten oder verwerfen
5) 700 µl Waschpuffer (vgl. 3.3.) auf die spin-Säule pipettieren und wie in 3) beschrieben, zentrifugieren, um kontaminierende Zellbestandteile zu entfernen
6) die membrangebundenen Nukleinsäuren zweimal mit 700 µl 80 % Ethanol in Wasser salzfrei waschen, hierbei vorgehen wie in 5)
7) die spin-Säule zwei Minuten bei maximaler Drehzahl zentrifugieren, um Ethanol vollständig zu entfernen
8) 50 bis 100 µl auf 80°C erwärmtes Wasser direkt auf die Membran der spin-Säule pipettieren und 1 Minute bei maximaler Drehzahl zentrifugieren, um die Nukleinsäuren zu eluieren; den Elutionsschritt gegebenenfalls wiederholen.

### 4.2. "batch-Prozedur"

1) Nukleinsäure enthaltende Quelle mit Lysepuffer (vgl. 3.1.) versetzen und mittels eines Handhomogenisators vollständig homogenisieren
2) Bindungsreagenz (vgl. 3.2.) zugeben, um die jeweiligen Bindebedingungen einzustellen
3) 50 µl Silica-Suspension (50 % im Lysepuffer) zugeben und zur Bindung der Nukleinsäuren 10 Minuten bei Raumtemperatur inkubieren, dabei mehrmals heftig aufwirbeln (vortexen)
4) 15 Sekunden bei 10.000 Upm in einer Tischzentrifuge zentrifugieren, um das Silica-Material zu pelletieren
5) den Überstand abpipettieren und gegebenenfalls weiteraufarbeiten oder verwerfen
6) 700 µl Waschpuffer (vgl. 3.3.) zum Pellet geben, heftig aufwirbeln (vortexen) bis das Pellet vollständig resuspendiert ist und wie in 4) zentrifugieren
7) Waschschritt 6) zweimal mit 700 µl 80 % Ethanol in Wasser wiederholen, um das Silica-Material salzfrei zu waschen
8) pelletiertes Silica-Material 10 Minuten bei 56°C mit offenem Deckel trocknen
9) 50 bis 100 µl Wasser zugeben, das Pellet durch heftiges Aufwirbeln (Vortexen) komplett resuspendieren und 10 Minuten bei 56°C inkubieren, dabei mehrmals heftig aufwirbeln (vortexen); diesen Elutionsschritt gegebenenfalls wiederholen
10) 1 Minute bei maximaler Drehzahl zentrifugieren und den Überstand in ein neues Reaktionsgefäß überführen.

### 5. Elektrophoretische Methoden

Die isolierten Nukleinsäuren wurden auf Ethidiumbromid-gefärbten Agarosegelen analysiert. Hierzu wurden 1,2 % Formaldehyd- oder 1,2 % 1 x TBE-Gele angefertigt.

Formaldehydgele wurden nach dem Lauf 3 bis 4 Stunden in Wasser, danach über Nacht in 10 µg/ml RNase A geschüttelt, um die RNA zu verdauen und somit die DNA besser sichtbar zu machen. TBE-Gele wurden ohne vorheriges Äquilibrieren RNase-verdaut.

Die im folgenden beschriebenen Beispiele verdeutlichen die Durchführung des erfindungsgemäßen Verfahrens. Alle hiernach isolierten Nukleinsäuren wurden elektrophoretisch analysiert und photometrisch quantifiziert. Der OD 260/280-Wert lag für alle Eluate zwischen 1.7 und 2.0.

### Referenzbeispiele 1 bis 5

### Isolierung von Gesamtnukleinsäure

In den nachfolgend aufgeführten Referenzbeispielen 1 bis 5 wurden die Binde-, Wasch- und Elutionsbedingungen jeweils so gewählt, daß sowohl DNA als auch RNA an den mineralischen Träger binden und gemeinsam eluiert werden.

Es wird anhand dieser Beispiel die Verwendung verschiedener Alkohole (Ethanol, Isopropanol, Butanol) als Bindungsreagenz verdeutlicht.

### Referenzbeispiel 1

### Isolierung von Gesamtnukleinsäure aus Nierengewebe

Aus 15 mg Nierengewebe (Ratte) wurde Gesamtnukleinsäure nach dem Standardprotokoll 4.1 isoliert. Das Gewebe wurde mit 400 µl L1 versetzt und homogenisiert, anschließend wurden 280 µl B1 zugegeben. Der ersten Waschschritt wurde mit W1 durchgeführt, das Elutionsvolumen betrug 2 x 50 µl.

### Referenzbeispiel 2

### Isolierung von Gesamtnukleinsäure aus Lebergewebe

Aus 7 mg Lebergewebe (Ratte) wurde Gesamtnukleinsäure nach dem Standardprotokoll 4.1 isoliert. Das Gewebe wurde mit 300 *µ*l L2 versetzt und homogenisiert, anschließend wurden 200 *µ*l B2 zugegeben. Der erste Waschschritt wurde mit W1 durchgeführt, das Elutionsvolumen betrug 2 x 50 µl.

### Referenzbeispiel 3

### Isolierung von Gesamtnukleinsäure aus HeLa-Zellen

Aus 1 x 10⁶ HeLa-Zellen wurde Gesamtnukleinsäure nach dem Standardprotokoll 4.1 isoliert. Die Zellen wurden mit 400 µl L2 versetzt und homogenisiert, anschließend wurden 200 µl B1 zugegeben. Der erste Waschschritt wurde mit W1 durchgeführt, das Elutionsvolumen betrug 1 x 50 µl.

### Referenzbeispiel 4

### Isolierung von Gesamtnukleinsäure aus Plasma

Gesamtnukleinsäure aus Plasma wurde parallel nach Standardprotokoll 4.1 und 4.2 isoliert. Hierfür wurden jeweils 200 µl Plasma mit 800 µl L3 und 660 µl B2 versetzt und gemischt; homogenisieren war hier nicht erforderlich. Im Ansatz für die "batch-Prozedur" (4.2) wurden zusätzlich 40 *µ*l Silica-Suspension zugegeben. Der erste Waschschritt wurde in beiden Ansätzen mit W2 durchgeführt, das Elutionsvolumen betrug 2 x 100 µl.

### Beispiel 1

### Fraktionierte Bindung von RNA und DNA bei konstanter GTC-Konzentration und steigender Ethanolkonzentration

Die Abhängigkeit der RNA/DNA-Bindung an den mineralischen Träger bei konstanter GTC- und steigender Ethanolkonzentration wurde gezeigt, indem je Probenansatz 10 mg eines Nierengewebes in 350 µl L4 lysiert und zur Einstellung der Ethanolkonzentration je Ansatz 350 µl eines Ethanol/Wasser-Gemisches zugegeben wurde, dessen Ethanolgehalt zwischen 20 und 90 % Ethanol in Wasser lag. Zu einem weiteren Ansatz wurden 350 µl absoluter Ethanol gegeben. Dies entsprach in den jeweiligen Ansätzen Bindebedingungen von konstanter GTC-Konzentration 1,75 M und steigender Ethanolkonzentration im Bereich von 10 bis 50 % (vgl. Fig. 1).

Zu den so eingestellten Lysaten wurden in einer ersten Versuchsreihe je Ansatz 150.000 cpm eines ³²P-markierten 0.9 kb in vitro Transkripts gegeben und das Lysat auf den in eier spin-Säule fixierten mineralischen Träger pipettiert. Es wurde 15 Sekunden bei 10.000 Upm in einer Tischzentrifuge zentrifugiert und die Menge der an die Säule gebundenen und der im Säulendurchbruch befindlichen Radioaktivität durch Cherenkov-Zählung bestimmt.

Die Versuchsreihe wurde wiederholt, wobei statt der ³²P-markierten RNA, 150.000 cpm eines durch Klenow-Auffüllreaktion ³²P-markierten, linearisierten pTZ-Plasmides zugegeben wurden.

Wie Fig. 1 zeigt, bindet die RNA-Fraktion unter den beschriebenen Bedingungen bereits ab Ethanolkonzentrationen größer 25 % an den mineralischen Träger, während die DNA-Fraktion erst ab Ethanolkonzentrationen größer 40 % bindet.

### Beispiele 2 bis 8

### Isolierung von Gesamt-RNA

In den folgenden Beispielen wurden zur Bindung an den mineralischen Träger die Alkohol/Salz-Gemische so gewählt (vgl. Fig. 1), daß eine selektive RNA-Bindung erreicht wurde.

Die Bindebedingungen wurden dabei auf die Art des jeweiligen Aufschlußmaterials (Gewebe, Zellkultur, Pflanzen, Bakterien) abgestimmt.

Die Beispiele verdeutlichen die verwendung von GTC, GuHCl oder GTC/Ethanol-Gemischen zur Lyse der Ausgangsmaterialien. Die Integrität der isolierten RNA wurde durch Northern-blotting oder RT-PCR verifiziert.

Auf die Aufarbeitung der nicht an den Träger gebundenen DNA wurde in diesen Beispiele verzichtet. Die Aufreinigung von DNA aus dem Säulendurchbruch wird in Beispiel 12 gezeigt. Darüber hinaus kann die DNA durch Einstellung der in den Referenzbeispielen 1 bis 5 gewählten Bindebedingungen aufgereinigt werden.

### Beispiel 2

### Isolierung von Gesamt-RNA aus Milzgewebe

Aus 15 mg Milzgewebe (Maus) wurde Gesamt-RNA nach dem Standardprotokoll 4.1 isoliert. Das Gewebe wurde mit 350 µl L4 versetzt und homogenisiert, anschließend wurden 350 µl B4 zugegeben. Der erste Waschschritt wurde mit W3 durchgeführt, das Elutionsvolumen betrug 1 x 50 µl.

### Beispiel 3

### Isolierung von Gesamt-RNA aus Lebergewebe (A)

In diesem Beispiel wurde ein ethanol-haltiger Lysepuffer verwendet, so daß das Standardprotokoll 4.1 leicht modifiziert wurde.

8 mg Lebergewebe (Ratte) wurden mit 700 µl L5 versetzt und homogenisiert. Das Lysat wurde auf die spin-Säule pipettiert und ab Schritt 3) das Standardprotokoll 4.1 durchgeführt. Der erste Waschschritt wurde mit W3 durchgeführt, das Elutionsvolumen betrug 1 x 50 µl.

### Beispiel 4

### Isolierung von Gesamt-RNA aus Lebergewebe (B)

Aus 15 mg Lebergewebe (Ratte) wurde Gesamt-RNA nach dem Standardprotokoll 4.1 isoliert. Das Gewebe wurde mit 300 µl L6 versetzt und homogenisiert, anschließend wurden 175 µl B1 zugegeben. Der erste Waschschritt wurde mit W4 durchgeführt, das Elutionsvolumen betrug 1 x 50 µl.

### Beispiel 5

### Isolierung von Gesamt-RNA aus HeLa-Zellen

Aus 1 x 10⁷ HeLa-Zellen wurde Gesamt-RNA parallel nach den Standardprotokollen 4.1 und 4.2 isoliert. Die Zellen wurden jeweils mit 350 µl L7 versetzt und homogenisiert, anschließend wurden je 350 µl B4 zugegeben. Im Ansatz für die "batch-Prozedur" (4.2) wurden zusätzlich 50 µl Silica-Suspension zugegeben. Der erste Waschschritt wurde mit W3 durchgeführt, das Elutionsvolumen betrug 1 x 50 µl.

### Beispiel 6

### Isolierung von Gesamt-RNA aus Tabak

Zur Gesamt-RNA-Isolierung aus Pflanzen wird das Standardprotokoll 4.1 leicht modifiziert. Nach Schritt 1) des Protokolls (Lyse), wird ein Zentrifugationsschritt bei 5.000 Upm in einer Tischzentrifuge eingefügt, um nicht lysierte Zellbestandteile, wie z.B. Faserreste, abzutrennen. Der Überstand wird abgenommen, mit Bindungsreagenz versetzt und ab Schritt 2) nach der Standardprozedur weiterverarbeitet.

Aus 100 mg Tabakblättern wurde Gesamt-RNA nach dem für Pflanzen modifizierten Standardprotokoll 4.1 isoliert. Das pulverisierte Zellmaterial wurde mit 600 µl L2 versetzt und homogenisiert, anschließend wurden 350 *µ*l B4 zugegeben. Der erste Waschschritt wurde mit W3 durchgeführt, das Elutionsvolumen betrug 1 x 50 µl.

### Beispiel 7

### Isolierung von Gesamt-RNA aus E.coli

Zur Isolierung von Gesamt-RNA aus Bakterien wird vor der Durchführung des Standardprotokolls ein zusätzlicher Arbeitsschritt eingefügt, um die Zellwände der Bakterien zu lysieren. Hierzu wird das Zellpellet in 400 µg/ml Lysozym in TE resuspendiert und 5 min auf Eis sowie 10 min bei Raumtemperatur inkubiert. Anschließend wird nach der Standardprozedur lysiert.

Aus 1 x 10⁹ E.coli wurde Gesamt-RNA nach dem für Bakterien modifizierten Standardprotokoll 4.1 isoliert. Das Pellet wurde in 80 µl 400 µl/ml Lysozym in TE resuspendiert und wie oben beschrieben inkubiert. Anschließend wurden 270 µl L2 zugegeben, homogenisiert und 350 µl B4 zugegeben. Der erste Waschschritt wurde mit W3 durchgeführt, das Elutionsvolumen betrug 2 x 50 µl.

### Beispiel 8

### Selektive RNA-Bindung durch Optimierung des Waschpuffers

Wie dieses Beispiel zeigt, können durch Optimierung des im ersten Waschschrittes verwendeten Waschpuffers (Standardprotokoll 4.1.5) DNA-Kontaminationen von der spezifisch gebundenen RNA entfernt werden.

1 x 10⁶ HeLa-Zellen wurden jeweils nach Standardprotokoll 4.1 in 350 µl L4 lysiert, mit 350 µl B4 versetzt und an den Silica-Träger gebunden. Die Proben wurden dann im ersten Waschschritt mit folgenden Waschpuffer gewaschen:

**Tab. 1**

| Zusammensetzung der Waschpuffer zum Auswaschen von DNA-Kontaminationen | | | |
|---|---|---|---|
| Probe | Waschpuffer | | |
| Nr. | M GTC | 25 mM TRIS/HCl pH 7.5 | % Ethanol |
| 1 | 0.3 | + | 5 |
| 2 | 0.6 | + | 5 |
| 3 | 0.9 | + | 5 |
| 4 | 0.3 | - | 5 |
| 5 | 0.6 | - | 5 |
| 6 | 0.9 | - | 5 |
| 7 - 12 | wie 1-6, jedoch 10 % EtOH | | |
| 8 - 18 | wie 1-6, jedoch 20 % EtOH | | |
| R*) | 1.75 | - | 35 |

| | | | |
|---|---|---|---|
| *) Die Probe diente als Referenz; die Zusammensetzung des Waschpuffers entsprach den Bindebedingungen | | | |

Die weiteren Arbeitsschritte erfolgten nach dem Standardprotokoll; das Elutionsvolumen betrug 1 x 50 µl.

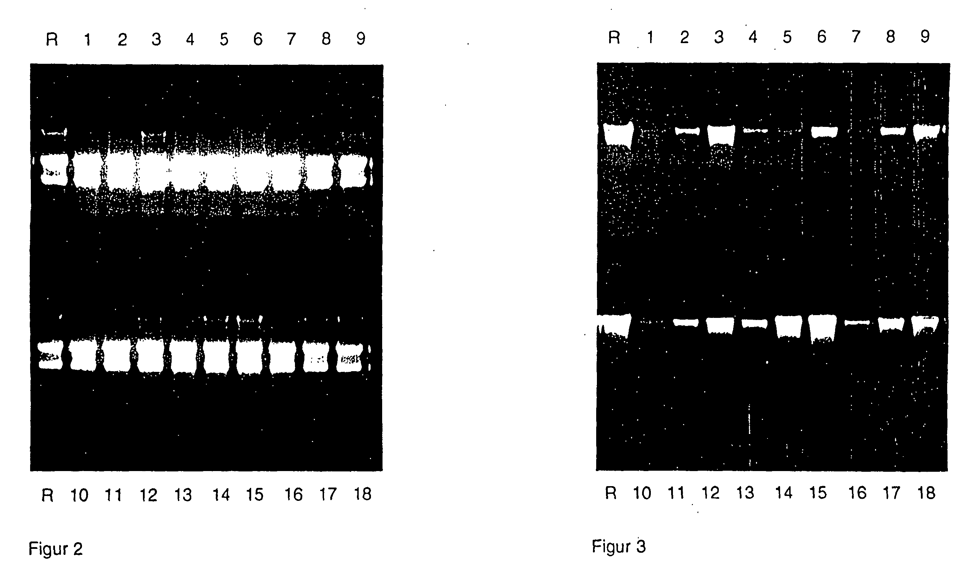

Die Hälfte des Eluates wurde auf einem 1,2 % Formaldehydgel analysiert (vgl. Fig. 2). Das Gel wurde anschließend wie unter "elektrophoretische Methoden" beschrieben mit RNaseA behandelt (vgl. Fig. 3).

### Legende zu Figuren 2 und 3

Fig. 2: 1,2 % Formaldehydgel zur Analyse der Eluate aus Beispiel 8, Bindebedingungen: 1,75 M GTC, 12.5 m;, Nacitrat pH 7.5, 35 % Ethanol; Waschbedingungen: vgl. Tab. 1. Die Benennung der Spuren entspricht der Bezeichnung der Proben in Tabelle 1.

### Fig. 3: RNase-Dau des Geles Fig. 2

### Beispiele 9 und 10

### Isolierung von DNA

In den nachfolgend aufgeführten Beispielen 9 und 10 wurden die Bindebedingungen so gewählt, daß nur die DNA an den mineralischen Träger binden kann, während die RNA durchbricht.

Auf die Aufarbeitung der nicht an den Träger gebundenen RNA wurde in diesen Beispielen verzichtet. Die Aufreinigung von RNA aus dem Säulendurchbruch wird in Beispiel 11 gezeigt. Darüberhinaus kann die RNA im Säulendurchbruch durch Einstellung der in Beispiel 2 bis 8 gewählten Bindebedingungen aufgereinigt werden.

Die selektive DNA-Bindung erfolgt im Lysepuffer in Abwesenheit von Alkohol, d.h. Schritt 2) der Standardprotokolle 4.1 und 4.2 entfällt.

### Beispiel 9

### Isolierung von genomischer DNA aus Nierengewebe

10 mg Nierengewebe (Ratte) wurde in 700 µl L8 lysiert. Die DNA wurde ohne Zugabe von Bindungsreagenz an den mineralischen Träger gebunden und im ersten Waschschritt mit 700 µl L8 gewaschen. Danach wurde das Standardprotokoll 4.1 ab Schritt 6) durchgeführt. Das Elutionsvolumen betrug 2 x 50 µl.

### Beispiel 10

### Isolierung von genomischer DNA aus HeLa-Zellen

1 x 10⁷ HeLa-Zellen wurden in 700 µl L9 lysiert. Die DNA wurde ohne Zugabe von Bindungsreagenz an den mineralischen Träger gebunden und im ersten Waschschritt mit 700 *µ*l L9 gewaschen. Danach wurde das Standardprotokoll 4.1 ab Schritt 6) durchgeführt. Das Elutionsvolumen betrug 2 x 50 µl.

### Beispiele 11 bis 13

### Trennung von Gesamt-RNA und genomischer DNA

Die nachfolgenden Beispiele 11 bis 13 zur getrennten Aufarbeitung von RNA und DNA aus demselben Zell-Lysat stellen Verknüpfungen der oben aufgeführten Beispiele zur RNA-, DNA- bzw. Gesamtnukleinsäureisolierung dar.

Die Trennung kann entweder durch differentielle Bindung oder durch fraktionierte Elution von RNA und DNA erfolgen.

### Beispiele 11 und 12

### Trennung von Gesamt-RNA und genomischer DNA durch differentielle Bindung

Zur differentielle Bindung gibt es wiederum zwei Möglichkeiten:

Nach der Lyse können die Bedingungen entweder so gewählt werden, daß zunächst die DNA an den mineralischen Träger bindet (Beispiel 11), oder aber die RNA kann im ersten Bindungsschritt adsorbiert werden, während die DNA aus dem Durchbruch aufgearbeitet wird (Beispiel 12).

### Beispiel 11

### Isolierung von genomischer DNA und Gesamt-RNA aus Nierengewebe

10 mg Nierengewebe (Ratte) wurden in 350 *µ*l L8 lysiert und die DNA im Lysepuffer an den mineralischen Träger gebunden. Zum Säulendurchbruch wurden 350 µl B4 gegeben und analog Beispiel 3.1 die Gesamt-RNA isoliert. Die Isolierung der genomischen DNA erfolgte wie in Referenzbeispiel 1.

### Beispiel 12

### Isolierung von Gesamt-RNA und genomischer DNA aus Lungengewebe

Aus 20 mg Lungengewebe (Ratte) wurde die Gesamt-RNA wie in Beispiel 2 beschrieben isoliert. Die nicht-gebundene genomische DNA im Säulendurchbruch wurde isoliert, indem 350 µl B1 und 350 µl B5 zugegeben wurden und die DNA wie in Standardprotokoll 4.1 beschreiben an den mineralischen Träger gebunden wurde. Der ersten Waschschritt wurde mit W1 durchgeführt, das Elutionsvolumen betrug 2 x 50 µl.

### Beispiel 13

### Trennung von Gesamt-RNA und genomischer DNA durch fraktionierte Elution

Das folgenden Beispiel verdeutlicht die selektive Elution der DNA-Fraktion der an den mineralischen Träger gebundenen Gesamtnukleinsäure.

Die Bindebedingungen werden so gewählt, daß die Gesamtnukleinsäure an den mineralischen Träger bindet. Die DNA-Fraktion wird anschließend eluiert, während die RNA-Fraktion gebunden bleibt. Die eluierte DNA wird durch erneutes Einstellen auf DNA-Bindebedingungen (vgl. Fig. 1) an einen weiteren mineralischen Träger gebunden und aufgearbeitet.

### Isolierung von genomischer DNA und Gesamt-RNA aus Lebergewebe

15 mg Lebergewebe (Schwein) wurden nach Standardprotokoll 4.1 1) bis 4) in 300 µl L2 lysiert, mit 250 µl B1 versetzt und die Gesamtnukleinsäure an den mineralischen Träger gebunden. Die DNA-Fraktion wurde mit 300 µl W5 eluiert, während das Trägermaterial mit der noch gebundenen RNA-Fraktion ab 5) nach dem Standardprotokoll 4.1 behandelt wurde. Die DNA-Fraktion wurde aus dem Eluat durch Zugabe von 350 µl B1 und 250 µl B5 und Bindung an einen weiteren mineralischen Träger nach dem Standardprotokoll 4.1 isoliert.

## Patentansprüche

1. Verfahren zur Trennung von doppel- und einzelsträngigen Nukleinsäuren aus diese Stoffe enthaltenden Quellen, wobei eine Probe mit mindestens einem mineralischen Träger behandelt wird, wobei
1.1 die Behandlungsbedingungen durch ein wässriges Gemisch von chaotropen Salzen in einer Konzentration von 1 bis 10 M und niederen aliphatischen Alkoholen mit 1 bis 5 Kohlenstoffatomen in einer Konzentration von 1 bis 90 Vol.-% so eingestellt sind, dass überwiegend die einzelsträngige Nukleinsäure enthaltende Fraktion an einem mineralischen Träger adsorbiert wird, während die doppelsträngige Nukleinsäure nicht adsorbiert wird, woraufhin die am mineralischen Träger adsorbierte einzelsträngige Nukleinsäure, nach gegebenenfalls durchgeführten Waschschritten, eluiert wird unter Bedingungen geringer Ionenstärke oder mit Wasser,
oder
1.2 die Behandlungsbedingungen so eingestellt sind, dass Erdalkali-Ionen komplexierende Substanzen in Abwesenheit von niederen aliphatischen Alkoholen mit 1 bis 5 Kohlenstoffatomen in der Lösung enthalten sind, die einzelsträngige Nukleinsäure unter diesen Behandlungsbedingungen nicht an einem mineralischen Träger adsorbiert wird und von der übrigen Probe abgetrennt wird, die doppelsträngige Nukleinsäure jedoch überwiegend an den mineralischen Träger bindet, woraufhin die doppelsträngige Nukleinsäure, nach gegebenenfalls durchgeführten Waschschritten, eluiert wird unter Bedingungen geringer Ionenstärke oder mit Wasser
oder
1.3 die Behandlungsbedingungen so eingestellt sind, dass Netz-, Wasch- oder Dispergiermittel in Abwesenheit von niederen aliphatischen Alkoholen mit 1 bis 5 Kohlenstoffatomen in der Lösung enthalten sind und die einzelsträngige Nukleinsäure unter diesen Behandlungsbedingungen nicht an einem mineralischen Träger adsorbiert wird und von der übrigen Probe abgetrennt wird, die doppelsträngige Nukleinsäure jedoch überwiegend an den mineralischen Träger bindet, woraufhin die doppelsträngige Nukleinsäure, nach gegebenenfalls durchgeführten Waschschritten, vom ersten mineralischen Träger eluiert wird unter Bedingungen geringer Ionenstärke oder mit Wasser
oder
1.4 die Behandlungsbedingungen durch ein entsprechendes wässriges Gemisch von chaotropen Salzen in einer Konzentration von 1 bis 10 M und niederen aliphatischen Alkoholen mit 1 bis 5 Kohlenstoffatomen in einer Konzentration von 1 bis 90 Vol.-% so eingestellt sind, dass die Gesamtnukleinsäure (einzel- oder doppelsträngige) an einem mineralischen Träger adsorbiert wird, gefolgt von einer Fraktionierung der an den Träger gebundenen einzel-/doppelsträngigen Nukleinsäuren durch Elution der einzel-/doppelsträngigen Nukleinsäuren vom mineralischen Träger durch selektive Elution der doppelsträngigen Nukleinsäure mittels Behandlung mit einer Lösung verminderter Ionenstärke und Konzentration von niederen aliphatischen Alkoholen mit 1 bis 5 Kohlenstoffatomen oder Elution der einzelsträngigen Nukleinsäure mittels einer Lösung enthaltend eine Erdalkali-Ionen komplexierende Substanz und/oder ein Netz-, Wasch- oder Dispergiermittel sowie eine oder mehrere Salzarten,
wobei die jeweils andere Nukleinsäure an dem mineralischen Träger gebunden bleibt und, nach gegebenenfalls durchgeführten Waschschritten, vom mineralischen Träger eluiert wird unter Bedingungen geringer Ionenstärke oder mit Wasser.

2. Verfahren nach Anspruch 1, wobei das System zur Lysierung der die Nukleinsäuren enthaltenden Quellen chaotrope Substanzen in einer Konzentration von 0,1 bis 10 M enthält.

3. Verfahren nach Anspruch 1 und/oder 2, wobei die Nukleinsäuren enthaltende Quelle mit einer Lösung versetzt wird, die Natriumperchlorat, Guanidiniumhydrochlorid, Guanidinium-iso-thiocyanat Guanidiniumthiocyanat, Natriumjodid, Kaliumjodid und/oder Kombinationen davon in einer Konzentration von 0,1 bis 10 M enthält.

4. Verfahren nach mindestens einem der Ansprüche 1 bis 3, wobei die Nukleinsäuren enthaltende Quelle mit einer Lösung versetzt wird, die Natriumchlorid, Lithiumchlorid, Kaliumchlorid, Natriumacetat, Magnesiumchlorid, Harnstoff und/ oder Kombinationen davon in einer Konzentration von 0,1 bis 10 M enthält.

5. Verfahren nach mindestens einem der Ansprüche 1 bis 4, wobei die Alkoholgruppen aufweisenden Substanzen niedere aliphatische Alkohole wie Methanol, Ethanol, Isopropanol, Butanol und Pentanol in einer Konzentration von 1 bis 90 Vol.-% sind und die Salze, wie NaCl, KCl, LiCl, MgCl₂, NaAc, in Konzentration von 1 bis 10 M vorliegen.

6. Verfahren nach mindestens einem der Ansprüche 1 bis 5, wobei der mineralische Träger aus porösen oder nicht porösen Metalloxiden oder Metallmischoxiden, Silicagel, Materialien, die überwiegend aus Glas bestehen, wie nicht modifizierten Glaspartikeln, Glasmehl, Quarz, Aluminiumoxid, Zeolithe, Titandioxid, Zirkondioxid aufgebaut ist mit einer Partikelgröße des mineralischen Trägermaterials von 0,1 µm bis 1.000 µm und einer Porengröße von 2 bis 1.000 µm, und das poröse oder nicht poröse Trägermaterial in Form loser Schüttungen vorliegen kann oder, die Trägermaterialien als Filterschichten ausgebildet sind in Form von Filterschichten aus Glas, Quarz oder Keramik und/oder einer Membran, in der Silicagel angeordnet ist und/oder als Partikel oder Fasern aus mineralischen Trägern und Geweben aus Quarz oder Glaswolle vorliegen sowie Latex-Partikel mit oder ohne funktionellen Gruppen sind oder Frittenmaterialien aus Polyethylen, Polypropylen, Polyvinyliden-fluorid, insbesondere ultra high molecular weight Polyethylen, high density Polyethylen sind.

7. Verfahren nach mindestens einem der Ansprüche 1 bis 6, wobei die biologische Quelle Zellkulturen, Gewebe jeder Art, Körperflüssigkeiten wie Blut, Plasma, Serum, Urin, Faeces, Mikroorganismen wie Bakterien, Viren wie Cytomegalie-Virus, HIV, Hepatitis B, Hepatitis C, Hepatitis-8-Virus, Pflanzen, Pflanzenteile, Embryonen, Keimlinge, Früchte ist oder die Nukleinsäuren enthaltende Probe Gemische sind, die nach enzymatischen Reaktionen wie in vitro Transkription und/oder cDNA-Synthese und/oder reverse Transkription mit anschließender PCR anfallen.

8. Verfahren nach mindestens einem der Ansprüche 1 bis 7, wobei die Zellen zunächst in einem wässrigen Lyse-System enthaltend chaotrope Substanzen oder andere Salze aufgeschlossen (lysiert) werden oder damit versetzt werden.

9. Verfahren nach mindestens einem der Ansprüche 1 bis 8, wobei jeweils die erhaltenen Fraktionen durch weitere chromatographische Schritte gereinigt werden.

10. Verfahren nach mindestens einem der Ansprüche 1 bis 9, wobei die Erdalkali-Ionen bindende Substanz Ethylendiamintetraessigsäure (EDTA) oder EGTA und das Netz-, Wasch- oder Dispergiermittel ein Sarkosinat ist.

11. Verfahren nach mindestens einem der Ansprüche 1 bis 10, wobei die einzelsträngige Nukleinsäure RNA und die doppelsträngige Nukleinsäure DNA ist.

12. Verwendung einer Lösung enthaltend 0,5 bis 8,0 M Guanidiniumthiocyanat und/oder Guanidiniumhydrochlorid, 1 bis 50% Ethanol und/oder Isopropanol in einem Verfahren nach einem der Ansprüche 1 bis 11.

13. Verwendung einer wässrigen Lösung enthaltend 0,1 bis 3 M Guanidin-thiocyanat und/oder Guanidinhydrochlorid, 1 bis 30% Ethanol und/oder Isopropanol als Puffer zum Auswaschen von an mineralische Träger gebundenen Nukleinsäuren.

14. Verwendung einer wässrigen Lösungen enthaltend 1 bis 5 M Guanidin-thiocyanat und/oder 1 bis 8 M Guanidinhydrochlorid, 0,1 bis 3% Sarkosyl als Puffer zum Binden von DNA an mineralische Träger.

15. Verwendung einer wässrigen Lösung enthaltend 1 bis 5 M Guanidiniumthiocyanat und/oder 1 bis 8 M Guanidiniumhydrochlorid, 5 mM bis 200 mM EDTA oder EGTA als Puffer zum Binden von DNA, doppelsträngiger Nukleinsäure an mineralischen Träger sowie als Puffer um einzelsträngige Nukleinsäure (RNA) nicht an mineralischen Träger zu binden.

16. Kit zur Durchführung des Verfahrens nach mindestens einem der Ansprüche 1 bis 11 mit mineralischem Träger, der in einem zum Durchfluss geeigneten Hohlkörper angeordnet ist, Lösungen, die in den Ansprüchen 12 bis 15 genannt sind sowie weiteren Hilfsstoffen und/oder Zubehör.

## Claims

1. A process for the separation of double-stranded and single-stranded nucleic acids from sources containing such materials wherein a sample is treated with at least one mineral support, wherein
1.1 the treatment conditions are adjusted with an aqueous mixture of chaotropic salts in a concentration of from 1 to 10 M and lower aliphatic alcohols having from 1 to 5 carbon atoms in a concentration of from 1 to 90% by volume in such a way that the fraction containing single-stranded nucleic acid is predominantly adsorbed on a mineral support whereas the double-stranded nucleic acid is not adsorbed, whereupon, following optionally performed washing steps, said single-stranded nucleic acid adsorbed on said mineral support is eluted under conditions of low ionic strength or with water; or
1.2 the treatment conditions are adjusted in such a way that materials complexing alkaline earth metal ions are contained in the solution in the absence of lower aliphatic alcohols having from 1 to 5 carbon atoms, the single-stranded nucleic acid is not adsorbed on a mineral support under these treatment conditions and is separated from the rest of the sample, the double-stranded nucleic acid, however, predominantly binds to said mineral support, whereupon, following optionally performed washing steps, said double-stranded nucleic acid is eluted under conditions of low ionic strength or with water; or
1.3 the treatment conditions are adjusted such that wetting, washing or dispersing agents are contained in the solution in the absence of lower aliphatic alcohols having from 1 to 5 carbon atoms, and the single-stranded nucleic acid is not adsorbed on a mineral support under such treatment conditions and is separated from the rest of the sample, the double-stranded nucleic acid, however, predominantly binds to said mineral support, whereupon, following optionally performed washing steps, said double-stranded nucleic acid is eluted from said first mineral support under conditions of low ionic strength or with water; or
1.4 the treatment conditions are adjusted with an appropriate aqueous mixture of chaotropic salts in a concentration of from 1 to 10 M and lower aliphatic alcohols having from 1 to 5 carbon atoms in a concentration of from 1 to 90% by volume in such a way that the whole nucleic acid (single-stranded or double-stranded) becomes adsorbed on a mineral support, followed by fractionation of said single-stranded/double-stranded nucleic acids bound to said support by eluting said single-stranded/double-stranded nucleic acids from said mineral support by selective elution of said double-stranded nucleic acid by treatment with a solution of reduced ionic strength and reduced concentration of lower aliphatic alcohols having from 1 to 5 carbon atoms, or elution of said single-stranded nucleic acid with a solution containing a material complexing alkaline earth metal ions and/or a wetting, washing or dispersing agent as well as one or more types of salts;
wherein the other nucleic acid, respectively, remains bound to said mineral support and, after optionally performed washing steps, is eluted from said mineral support under conditions of low ionic strength or with water.

2. The process according to claim 1, wherein the system for lysing said sources containing the nucleic acids contains chaotropic substances in concentrations of from 0.1 to 10 M.

3. The process according to claim 1 and/or 2, wherein said source containing the nucleic acids is mixed with a solution containing sodium perchlorate, guanidinium chloride, guanidinium isothiocyanate/guanidinium thiocyanate, sodium iodide, potassium iodide, and/or combinations thereof in concentrations of from 0.1 to 10 M.

4. The process according to at least one of claims 1 to 3, wherein said source containing the nucleic acids is mixed with a solution containing sodium chloride, lithium chloride, potassium chloride, sodium acetate, magnesium chloride, urea, and/or combinations thereof in concentrations of from 0.1 to 10 M.

5. The process according to at least one of claims 1 to 4, wherein said materials containing alcohol groups are lower aliphatic alcohols, such as methanol, ethanol, isopropanol, butanol and pentanol, in concentrations of from 1 to 90% by volume, and said salts, such as NaCl, KCl, LiCl, MgCl₂, NaAc, are present in concentrations of from 1 to 10 M.

6. The process according to at least one of claims 1 to 5, wherein said mineral support consists of porous or non-porous metal oxides or mixed metal oxides, silica gel, materials predominantly consisting of glass, such as unmodified glass particles, powdered glass, quartz, alumina, zeolites, titanium dioxide, zirconium dioxide, the particle size of the mineral support material being from 0.1 µm to 1000 µm and the pore size being from 2 to 1000 µm, and said porous or non-porous support material may be present in the form of loose packings or said support materials are embodied in the form of filter layers made of glass, quartz or ceramics, and/or a membrane in which silica gel is arranged, and/or particles or fibers made of mineral supports and fabrics of quartz or glass wool, as well as latex particles with or without functional groups, or frit materials made of polyethylene, polypropylene, polyvinylidene fluoride, especially ultra high molecular weight polyethylene, high density polyethylene.

7. The process according to at least one of claims 1 to 6, wherein said biological source is cell cultures, all kinds of tissues, body fluids, such as blood, plasma, serum, urine, faeces; microorganisms, such as bacteria, viruses, such as cytomegalovirus, HIV, hepatitis B, hepatitis C, hepatitis δ virus; plants, plant parts, embryos, germs, fruits, or the samples containing nucleic acids are mixtures obtained from enzymatic reactions, such as in vitro transcription and/or cDNA synthesis and/or reverse transcription with subsequent PCR.

8. The process according to at least one of claims 1 to 7, wherein the cells are first lysed in an aqueous lysis system containing chaotropic substances or other salts, or mixed therewith.

9. The process according to at least one of claims 1 to 8, wherein the fractions obtained are respectively purified by further chromatographic steps.

10. The process according to at least one of claims 1 to 9, wherein said substance binding alkaline earth metal ions is ethylenediaminetetraacetic acid (EDTA) or EGTA, and said wetting, washing or dispersing agent is a sarcosinate.

11. The process according to at least one of claims 1 to 10, wherein said single-stranded nucleic acid is RNA and said double-stranded nucleic acid is DNA.

12. Use of a solution containing from 0.5 to 8.0 M guanidinium thiocyanate and/or guanidinium chloride, from 1 to 50% ethanol and/or isopropanol in a process according to any of claims 1 to 11.

13. Use of an aqueous solution containing from 0.1 to 3 M guanidine thiocyanate and/or guanidine hydrochloride, from 1 to 30% ethanol and/or isopropanol as a buffer for washing out nucleic acids bound to mineral supports.

14. Use of an aqueous solution containing from 1 to 5 M guanidine thiocyanate and/or from 1 to 8 M guanidine hydrochloride, from 0.1 to 3% sarcosyl as a buffer for binding DNA to mineral supports.

15. Use of an aqueous solution containing from 1 to 5 M guanidinium thiocyanate and/or from 1 to 8 M guanidinium hydrochloride, from 5 mM to 200 mM EDTA or EGTA as a buffer for binding DNA, double-stranded nucleic acid to mineral supports, and as a buffer for not binding single-stranded nucleic acid (RNA) to mineral supports.

16. A kit for performing the process according to at least one of claims 1 to 11 comprising a mineral support which is arranged in a hollow body suited for flow-through, solutions as mentioned in claims 12 to 15, as well as other auxiliaries and/or accessories.

## Revendications

1. Procédé de séparation d'acides nucléiques double et simple brin à partir de sources contenant ces substances, où un échantillon est traité avec au moins un support minéral, où
1.1 les conditions de traitement sont ajustées par un mélange aqueux de sels chaotropes en une concentration de 1 à 10 M et d'alcools aliphatiques inférieure avec 1 à 5 atomes de carbone en une concentration de 1 à 90% en volume, de sorte que la fraction contenant l'acide nucléique simple brin est essentiellement adsorbée sur un support minéral, alors que l'acide nucléique double brin n'est pas adsorbé, suite à quoi l'acide nucléique simple brin adsorbé sur le support minéral est élué, après une étape de lavage facultative, dans des conditions de faible force ionique ou avec de l'eau,
ou
1.2 les conditions de traitement sont ajustées de sorte que des substances complexant des ions alcalino-terreux sont présentes en solution, en l'absence d'alcools aliphatiques inférieurs ayant 1 à 5 atomes de carbone, l'acide nucléique simple brin n'est pas adsorbé sur un support minéral dans ces conditions de traitement et n'est pas séparé de l'échantillon restant, l'acide nucléique double brin se lie cependant essentiellement sur le support minéral, suite à quoi l'acide nucléique double brin est élué, après une étape de lavage facultative, dans des conditions de faible force ionique ou avec de l'eau,
ou
1.3 les conditions de traitement sont ajustées de sorte que des agents mouillants, de lavage ou dispersants sont présents en solution, en l'absence d'alcools aliphatiques inférieurs ayant 1 à 5 atomes de carbone, l'acide nucléique simple brin n'est pas adsorbé sur un support minéral dans ces conditions de traitement et n'est pas séparé de l'échantillon restant, l'acide nucléique double brin se lie cependant essentiellement sur le support minéral, suite à quoi l'acide nucléique double brin est élué du support minéral, après une étape de lavage facultative, dans des conditions de faible force ionique ou avec de l'eau,
ou
1.4 les conditions de traitement sont ajustées par un mélange aqueux approprié de sels chaotropes en une concentration de 1 à 10 M et d'alcools aliphatiques inférieurs avec 1 à 5 atomes de carbone en une concentration de 1 à 90% en volume, de sorte que tout l'acide nucléique (simple brin et double brin) est adsorbé sur un support minéral, puis on réalise un fractionnement de l'acide nucléique simple brin/double brin lié sur le support minéral, par élution sélective de l'acide nucléique double brin par traitement avec une solution de faible force ionique et faible concentration d'alcools aliphatiques inférieurs ayant 1 à 5 atomes de carbone ou élution de l'acide nucléique simple brin à l'aide d'une solution contenant une substance complexant les ions alcalino-terreux et/ou des agents mouillants, de lavage ou dispersants ainsi qu'un ou plusieurs sels,
où chaque fois, l'autre acide nucléique reste sur le support minéral et après une étape de lavage facultative, est élué du support minéral dans des conditions de faible force ionique ou avec de l'eau.

2. Procédé selon la revendication 1, où le système pour la lyse de la source contenant les acides nucléiques contient des substances chaotropes en une concentration de 0,1 à 10 M.

3. Procédé selon la revendication 1 ou 2, où on ajoute à la source contenant des acides nucléiques, une solution, qui contient du perchlorate de sodium, du chlorhydrate de guanidinium, de l'isothiocyanate de guanidinium, du thiocyanate de guanidinium, de l'iodure de sodium, de l'iodure de potassium et/ou une combinaison de ceux-ci en une concentration de 0,1 à 10 M.

4. Procédé selon au moins une des revendications 1 à 3, où on ajoute à la source contenant des acides nucléiques, une solution qui contient du chlorure de sodium, du chlorure de lithium, du chlorure de potassium, de l'acétate de sodium, du chlorure de magnésium, de l'urée et/ou une combinaison de ceux-ci en une concentration de 0,1 à 10 M.

5. Procédé selon au moins une des revendications 1 à 4, où les substances présentant des groupes alcool sont des alcools aliphatiques inférieurs comme le méthanol, l'éthanol, l'isopropanol, le butanol ou le pentanol en une concentration de 1 à 90% en volume et les sels, comme NaCl, KCl, LiCl, MgCl₂, NaAc, sont présents en une concentration de 1 à 10 M.

6. Procédé selon au moins une des revendications 1 à 5, où le support minéral est élaboré à partir d'oxydes métalliques ou d'oxydes métalliques mixtes poreux ou non poreux, de gel de silice, de matériaux qui consistent essentiellement en du verre, comme des particules en verre non modifié, de la poudre de verre, du quartz, de l'oxyde d'aluminium, une zéolite, le dioxyde de titane, le dioxyde de zirconium, avec une taille des particules du matériau de support minéral de 0,1 µm à 1000 µm et une taille des pores de 2 à 1000 µm, et le matériau support poreux ou non poreux peut se présenter sous forme libre ou, les matériaux supports ont la forme de couches filtre, en forme de couches filtre en verre, en quartz ou céramique et/ou d'une membrane, dans laquelle est disposé du gel de silice et/ou comme des particules ou fibres en le support minéral et des tissus en quartz ou laine de verre, ainsi que des particules de latex avec ou sans groupes fonctionnels ou des matériaux frittés en polyéthylène, polypropylène, poly(fluorure de vinylidène), en particulier du polyéthylène de poids moléculaire ultra élevé, du polyéthylène de haute densité.

7. Procédé selon au moins une des revendications 1 à 6, où la source biologique est des cultures cellulaires, des tissus de tout type, des fluides corporels comme le sang, le plasma, le sérum, l'urine, les fèces, des microorganismes comme des bactéries, des virus comme le cytomégalovirus, le HIV, les virus de l'hépatite B, de l'hépatite C, de l'hépatite δ, des plantes, des parties de plantes, des embryons, des germes, des fruits ou les échantillons contenant des acides nucléiques sont des mélanges, qui sont produits par des réactions enzymatiques comme la transcription *in vitro* et/ou la synthèse d'ADNc et/ou la transcription réverse avec PCR ultérieure.

8. Procédé selon au moins une des revendications 1 à 7, où les cellules sont d'abord détruites (lysées) ou ajoutées à un système aqueux de lyse contenant des substances chaotropes ou d'autres sels.

9. Procédé selon au moins une des revendications 1 à 8, où les fractions obtenues sont chaque fois purifiées par une autre étape de chromatographie.

10. Procédé selon au moins une des revendications 1 à 9, où les substances liant des ions alcalino-terreux sont l'acide éthylènediaminetétraacétique (EDTA) ou l'EGTA et l'agent mouillant, de lavage ou dispersant est un sarcosinate.

11. Procédé selon au moins une des revendications 1 à 10, où l'acide nucléique simple brin est un ARN et l'acide nucléique double brin est un ADN.

12. Utilisation d'une solution contenant 0,5 à 8,0 M de thiocyanate de guanidinium et/ou de chlorhydrate de guanidinium, 1 à 50% d'éthanol et/ou d'isopropanol dans un procédé selon une des revendications 1 à 11.

13. Utilisation d'une solution aqueuse contenant 0,1 à 3 M de thiocyanate de guanidinium et/ou de chlorhydrate de guanidinium, 1 à 30% d'éthanol et/ou d'isopropanol comme tampon pour le lavage d'acides nucléiques liés sur un support minéral.

14. Utilisation d'une solution aqueuse contenant 1 à 5 M de thiocyanate de guanidinium et/ou 1 à 8 M de chlorhydrate de guanidinium, 0,1 à 3% de sarcosyle comme tampon pour lier l'ADN sur un support minéral.

15. Utilisation d'une solution aqueuse contenant 1 à 5 M de thiocyanate de guanidinium et/ou 1 à 8 M de chlorhydrate de guanidinium, 5 mM à 200 mM d'EDTA ou d'EGTA comme tampon pour lier l'ADN, un acide nucléique double brin sur un support minéral, ainsi que comme tampon pour ne pas lier l'acide nucléique simple brin (ARN) sur un support minéral.

16. Kit pour réaliser le procédé selon au moins une des revendications 1 à 11, avec un support minéral, qui est disposé dans un corps creux approprié pour un écoulement, les solutions, qui sont citées dans les revendications 12 à 15, ainsi que d'autres auxiliaires et/ou accessoires.
